(19) Europäisches Patentamt
European Patent Office
Office européen des brevets

(11) **EP 1 518 579 B1**

(12) **EUROPÄISCHE PATENTSCHRIFT**

(45) Veröffentlichungstag und Bekanntmachung des Hinweises auf die Patenterteilung:
**28.11.2018 Patentblatt 2018/48**

(21) Anmeldenummer: **04016625.8**

(22) Anmeldetag: **15.07.2004**

(51) Int Cl.:
***A61M 16/00*** *(2006.01)*

(54) **Vorrichtung zur Beatmung**

Breathing device

Appareil respiratoire

(84) Benannte Vertragsstaaten:
**AT BE BG CH CY CZ DE DK EE ES FI FR GB GR HU IE IT LI LU MC NL PL PT RO SE SI SK TR**

(30) Priorität: **20.09.2003 DE 10343610**

(43) Veröffentlichungstag der Anmeldung:
**30.03.2005 Patentblatt 2005/13**

(73) Patentinhaber: **Löwenstein Medical Technology S.A.**
**2557 Luxembourg (LU)**

(72) Erfinder:
• **Köhler, Dieter, Prof. Dr.**
**57392 Schmallenberg (DE)**

• **Göbel, Christof, Dr.**
**22529 Hamburg (DE)**
• **Tiemann, Björn**
**22926 Ahrensburg (DE)**

(74) Vertreter: **Klickow & Wetzel PartGmbB**
**Jessenstraße 4**
**22767 Hamburg (DE)**

(56) Entgegenhaltungen:
**EP-A- 1 129 742      EP-A- 1 346 743**
**WO-A-01/00265      DE-A1- 10 014 427**
**US-B1- 6 349 724**

**Beschreibung**

Die Erfindung betrifft eine Vorrichtung zur Beatmung, durch den beigefügten Anspruch 1 definiert ist, die eine Atemgaspumpe, eine Steuereinheit für die Atemgaspumpe sowie einen Anschluss zur Verbindung mit einem Beatmungsschlauch aufweist und bei der die Steuereinheit mit einem Sollwertspeicher für mindestens einen Steuerparameter gekoppelt ist, der zu einem Beatmungsmuster korrespondiert, wobei die Steuereinheit mit einem Analysator gekoppelt ist, der mit mindestens einem Sensor für mindestens einen Atemparameter verbunden ist, und bei der die Steuereinheit einen Transformator zur Umrechnung von Daten des Analysators in Vorgabewerte für den Sollwertspeicher aufweist und bei der die Steuereinheit einen Koordinator zur Aktivierung einer Ansteuerung der Atemgaspumpe in Abhängigkeit von den errechneten und den im Sollwertspeicher abgelegten Steuerwerten einer Drucksteuerung aufweist.

Komfortable moderne Beatmungsgeräte ermöglichen es häufig, sowohl eine assistierte, eine mandatorische als auch eine gemischt assistierte und mandatorische, kontrollierte Beatmung durchzuführen. Eine assistierte Beatmung, bei der der Beatmungszyklus des Beatmungsgerätes in Abhängigkeit von einer messtechnisch erfassten eigenen Atmungsaktivität des Patienten gesteuert wird, weist den Vorteil auf, dass der Patient die Durchführung der Beatmung als angenehm und nur wenig störend empfindet. Es erfolgt jedoch nur eine teilweise Entlastung des Patienten von der durchzuführenden Atemarbeit. Bei einer mandatorischen Beatmung erfolgt eine weitgehende Entlastung des Patienten von der Atemarbeit, der Patient empfindet eine derartige Atmung - speziell in der Anfangsphase einer Behandlung - jedoch als ausgesprochen störend und unangenehm. Häufig wird ebenfalls beobachtet, dass der Patient versucht, trotz einer mandatorischen Beatmung seinen eigenen Atmungsrhythmus gegen das Beatmungsgerät durchzusetzen. Eine derartige Betriebsweise führt zu einer zusätzlich erhöhten Atemarbeit des Patienten.

Aus der EP-A-1 346 743 sind ein Verfahren zur Steuerung eines Beatmungsgerätes sowie eine Vorrichtung zur Beatmung bekannt, bei der ein Druckaufbau in Abhängigkeit von einem zu einem Messsignal korrespondierenden Trägersignal durchgeführt wird. Das Trägersignal wird mit einem Bezugswert verglichen. Der Bezugswert ist unter Verwendung eines Bezugswertadapters variabel.

Aus der EP-A-1 129 742 ist eine Vorrichtung zur Beatmung mit einer Atemgaspumpe bekannt. Die Atemgaspumpe wird von einer Steuereinheit geregelt, wobei die Steuereinheit einen Sollwertspeicher für einen Steuerparameter aufweist. Ein Atemparameter wird von einem Sensor erfasst und das entsprechende Signal einem Analysator zugeführt. Die Atemgaspumpe wird in Abhängigkeit von errechneten Steuerwerten aktiviert.

In der WO-A-01/00265 werden ein medizinisches Beatmungsgerät sowie ein Steuerungsverfahren für dieses Gerät beschrieben. Die Atemluft wird von einem Ventilator bereitgestellt, dem unterschiedliche Steuersätze zugeordnet sind. In Abhängigkeit von messtechnisch erfassten Parametern wird ein geeigneter Steuersatz ausgewählt.

In der US-B-6,349,724 wird ein Beatmungsgerät beschrieben, dass zwei unterschiedliche Druckpegel bereitstellt. Über Sensoren werden Beatmungsparameter eines Patienten erfaßt und einer Regelung zugeführt. Die Regelung führt eine Steuerung des Beatmungsgerätes durch und stellt darüber hinaus physiologische Daten zur Durchführung von Diagnosen und Therapien zur Verfügung.

Aus der DE 10014427 A1 ist bereits eine Vorrichtung zur Beatmung bekannt, die eine Atemgaspumpe und eine Steuereinheit für die Atemgaspumpe aufweist. Darüber hinaus ist ein Anschluss zur Verbindung mit einem Beatmungsschlauch vorgesehen. Die Steuereinheit ist mit einem Sollwertspeicher für mindestens einen Steuerparameter gekoppelt. Darüber hinaus ist die Steuereinheit mit einem Analysator gekoppelt.

Zu einer Synchronisation des Betriebes des Beatmungsgerätes mit einem natürlichen Atmungsrhythmus des Patienten bei der assistierten Beatmung ist es bereits bekannt, messtechnisch den Atemantrieb des Patienten zu erfassen und als Triggersignal für das Beatmungsgerät zu verwenden, um einen Druckaufbau bzw. einem Volumenaufbau auszulösen. Bei einer derartigen Beatmung verbraucht der Patient in der Regel 60 % des aufgenommenen Sauerstoffs in der Atemmuskulatur im Vergleich zu einer spontanen Atmung, da trotz Triggerung unterschwellig der Atemzyklus mitläuft.

Bei Patienten mit ventilatorischer Insuffizienz erweist es sich als vorteilhaft, eine möglichst weitgehende muskuläre Entlastung zu realisieren. Im Hinblick auf dieses Ziel erweist sich eine vollständige mandatorische Steuerung des Beatmungsgerätes als vorteilhaft gegenüber einer assistierten Gerätesteuerung. Bei einer Gerätesteuerung derart, dass eine gut angepasste und kontrollierte Beatmung realisiert wird, ist es möglich, den Sauerstoffverbrauch der Inspirationsmukulatur des Patienten um bis zu 90 % zu senken. Der entsprechende Sauerstoffverbrauch korreliert direkt zur geleisteten biologischen Atemarbeit des Patienten. Selbst bei empfindlicher und optimal angepaßter Triggerung lassen sich mit assistierten Beatmungsformen derzeit nur Entlastungen von maximal 40 % erreichen.

Dem Vorteil der muskulären Entlastung bei einer mandatorischen Gerätesteuerung steht der Nachteil entgegen, dass häufig nur eine geringe Akzeptanz durch den Patienten vorliegt. Für die Patienten stellt es insbesondere ein erhebliches Problem dar, den eigenen Atemantrieb zu unterdrücken. Die Akzeptanz einer kontrollierten Beatmung durch den Patienten ist umso geringer, je geringer ein durch die Gerätesteuerung realisiertes Druckprofil sowie Flowprofil auf einen aktuellen Bedarf des Patienten abgestimmt ist. Darüber hinaus erweist es sich als zusätzlich nachteilig, wenn beispielsweise eine Phasenlage sowie das zeitliche Verhältnis von Inspirationsphasen und Expirationsphasen deutlich von einem aktuell vom Patienten gewollten Beatmungsmuster abweichen.

Aufgabe der vorliegenden Erfindung ist es, eine Vorrichtung der einleitend genannten Art derart zu konstruieren, dass eine erhöhte Akzeptanz durch den Patienten unterstützt wird.

Diese Aufgabe wird erfindungsgemäß dadurch gelöst, dass der Analysator den Flowverlauf und die Atemfrequenz und das Verhältnis von Inspirationsphasen zu Expirationsphasen analysiert und dass daraus der Steuerwert als Druckmuster berechnet wird, sowie dass die Steuereinheit zur automatischen Einleitung einer Analysephase während der Durchführung der mandatorischen Beatmung als Reaktion auf eine Spontanatmung des Patienten ausgebildet ist.

Aufgrund der Durchführung einer Analysephase sowie der adaptiven Anpassung der Steuerparameter an die Analyseergebnisse ist es möglich, eine weitgehende Annäherung des steuerungstechnisch vorgegebenen mandatorischen Beatmungsmusters an ein individuelles und aktuelles Spontanatmungsmuster des Patienten vorzunehmen. Dies hat zur Folge, dass zwar einerseits eine vollständig mandatorische und vom Patienten entkoppelte Gerätesteuerung realisiert wird, die den Patienten weitgehend von einer eigenen Atemarbeit entlastet, dass andererseits aber eine individuelle Adaption des gerätetechnisch vorgegebenen Beatmungsmusters erfolgt,
so daß der Patient die durchgeführte Beatmung nicht als aufgezwungen und somit als unangenehm empfindet. Bei einer Durchführung des Verfahrens bzw. bei einer Benutzung der Vorrichtung können somit die Vorteile einer mandatorischen Beatmung und einer assistierten Beatmung miteinander kombiniert werden, ohne daß die jeweiligen Nachteile wirksam werden.

Ein typischer Verfahrensablauf erfolgt derart, daß eine Analyse eines Flowmusters durchgeführt wird. Unter einem Muster werden hierbei zyklisch wiederkehrende Signalverläufe verstanden.

Beispielsweise ist es möglich, daß die Analysephase bei einem einwirkenden Umgebungsdruck durchgeführt wird.

Darüber hinaus ist auch daran gedacht, daß die Analysephase bei einem einwirkenden kontinuierlich positivem Atemwegsdruck durchgeführt wird.

Weiterhin ist es auch möglich, daß die Analysephase bei assistierter Beatmung durchgeführt wird, wobei der kontinuierlich positive Atemwegsdruck inspiratorisch größer ist als der expiratorische Druck.

Gemäß einer alternativen Verfahrensrealisierung ist es auch möglich, daß eine Analyse eines Flowverlaufes durchgeführt wird.

Eine Analysevariante besteht darin, daß eine Analyse einer Atemfrequenz durchgeführt wird.

Ebenfalls ist es möglich, daß eine Analyse eines Verhältnisses von Inspirationsphasen zu Expirationsphasen durchgeführt wird.

Andere Varianten bestehen darin, daß eine Analyse eines Maximum-Flows durchgeführt wird, oder daß eine Analyse eines Volumenverlaufes durchgeführt wird.

Darüber hinaus ist es auch möglich, daß eine Analyse eines Tidalvolumens durchgeführt wird, oder daß eine Analyse des Atmungswiderstandes der Atemwege durchgeführt wird, beziehungsweise daß eine Analyse des Strömungsverhaltens der Atemwege durchgeführt wird.

Gemäß weiterer Ausführungsformen ist vorgesehen, daß eine Analyse einer intrinsischen PEEP durchgeführt wird, oder daß eine Analyse eines ETCO2 durchgeführt wird, oder daß eine Analyse eines SPO2 durchgeführt wird.

Schließlich ist es auch möglich, daß eine Analyse der Körperlage des Patienten durchgeführt wird, oder daß eine Analyse einer Toraxbewegung durchgeführt wird, oder daß eine Analyse einer Abdomenbewegung durchgeführt wird.

Zur Realisierung von komplexeren Analyseverfahren ist daran gedacht, daß eine Analyse eines EKG-Verlaufs durchgeführt wird, oder daß eine Analyse eines EEG-Verlaufs durchgeführt wird, oder daß eine Analyse eines EMG-Verlaufs durchgeführt wird.

Eine vereinfachte Transformation der Analysewerte in Sollwerte kann dadurch erfolgen, daß das Beatmungsmuster als Sollwert entsprechend der Gleichung

$$P(t) = Resistance * Flow(t) + Elastance * Volumen(t) + PEEPintr.$$

berechnet wird.

Bei der Transformation des aufgenommenen Spontanmusters in ein Beatmungsmuster unter Verwendung der Bewegungsgleichung ist es erforderlich, Informationen hinsichtlich der Resistance, der Compliance und des intrinsischen PEEP zu besitzen. Diese Größen können entweder durch eine externe Dateneingabe definiert oder durch ein spezielles Meßverfahren, beispielsweise der Okklusionsmethode (kompletter oder teilweiser Verschluß), erfaßt werden. Von Bedeutung ist insbesondere auch die Erfassung der sogenannten Elastance, worunter die elastischen Rückstellkräfte von Lunge bzw. Brustraum des Patienten zu verstehen sind.

Zur Unterstützung von komplexeren Auswertungsalgorhythmen ist daran gedacht, daß der Steuerparameter von einem Expertensystem berechnet wird.

Gemäß einer Ausführungsvariante ist es möglich, daß der Steuerwert als Druckmuster berechnet wird.

Gemäß einer anderen Ausführungsvariante ist vorgesehen, daß der Steuerwert als Flowmuster berechnet wird.

Eine besonders hohe Akzeptanz durch den Patienten kann dadurch erreicht werden, daß ein Übergang zu einer mandatorischen Gerätesteuerung in Abhängigkeit vom Steuerparameter während einer Übergangsphase durchgeführt wird.

Ein kontinuierlicher Übergang zu einer vollständigen mandatorischen Steuerung kann beispielsweise dadurch erfolgen, daß während der Übergangsphase ein langsamer Anstieg des maximalen Druckes je Atemzug von Atemzug zu Atemzug erfolgt.

Eine andere Ausführungsvariante besteht darin, daß eine Mischung des Sollwertmusters mit einem Standardmuster durchgeführt wird.

Eine exakte Realisierung eines vorgegebenen Beatmungsmusters kann beispielsweise dadurch erfolgen, daß von der Gerätesteuerung Form, Takt und Geschwindigkeit des Beatmungsmusters vorgegeben werden.

Eine kurzfristige Adaption der Steuerung wird dadurch unterstützt, daß ein Übergang von einer mandatorischen Gerätesteuerung zu einer Analysephase bei einer Detektion einer regelmäßigen Spontanatmung durchgeführt wird.

Eine weitere Adaptionsvariante besteht darin, daß ein Übergang von einer Analysephase zu einer mandatorischen Gerätesteuerung bei einer Detektion einer Patienteneigenaktivität oberhalb eines vorgegebenen Schwellwertes durchgeführt wird.

Eine weitere Ausführungsvariante betrifft die Unterstützung einer Targetventilation. Es werden hierbei extern Sollwerte für die Durchführung der Beatmung hinsichtlich der Beatmungsfrequenz sowie des Atemzeitverhältnisses vorgegeben. Das Beatmungsgerät wird hierbei zunächst entsprechend der Spontanfrequenz des Patienten oder einem leicht darüber liegenden Wert betrieben, anschließend erfolgt eine Anpassung der Beatmungssteuerung langsam und unter Überwachung der Eigenaktivität des Patienten in Richtung auf die Sollfrequenz.

Wird beim Absenken der Beatmungsfrequenz erneut eine Eigenaktivität des Patienten meßtechnisch erfaßt, so erfolgt eine Rückkopplung hinsichtlich der Gerätesteuerung derart, daß eine Korrektur der Beatmungsfrequenz in Richtung auf die ursprüngliche Spontanfrequenz erfolgt. Hierdurch wird eine Prioritätssteuerung derart realisiert, daß einer Vermeidung von Eigenaktivitäten des Patienten eine höhere Priorität als einem Erreichen des Sollwertes eingeräumt wird und daß von der Gerätesteuerung diese Prioritäten bei der Vorgabe einer aktuellen Gerätesteuerung berücksichtigt werden.

In den Zeichnungen sind Ausführungsbeispiele der Erfindung schematisch dargestellt. Es zeigen:

Fig. 1    Eine perspektivische Darstellung eines Beamtungsgerätes mit Verbindungsschlauch zu einer Beatmungsmaske und

Fig. 2    ein schematisches Blockschaltbild zur Veranschaulichung des Aufbaues der Vorrichtung.

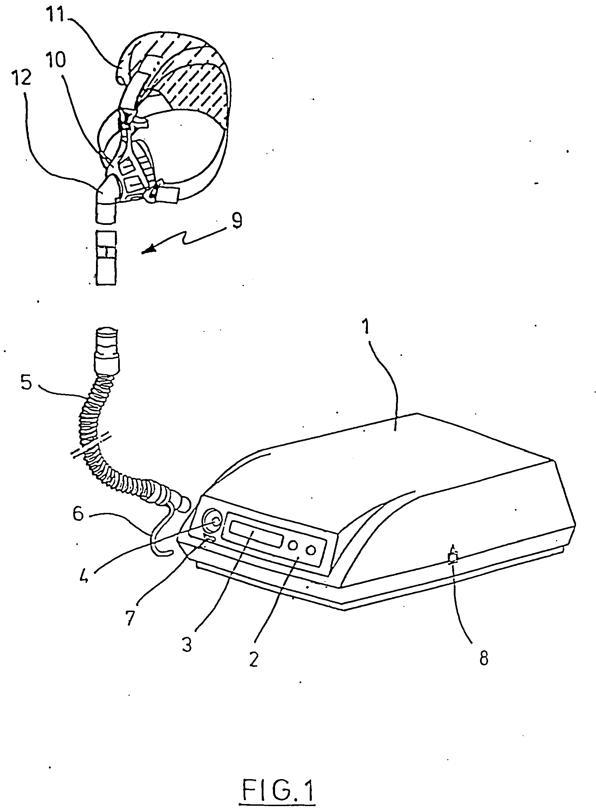

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Gemäß einer anderen Ausführungsform kann auch eine Vollgesichtsmaske verwendet werden. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

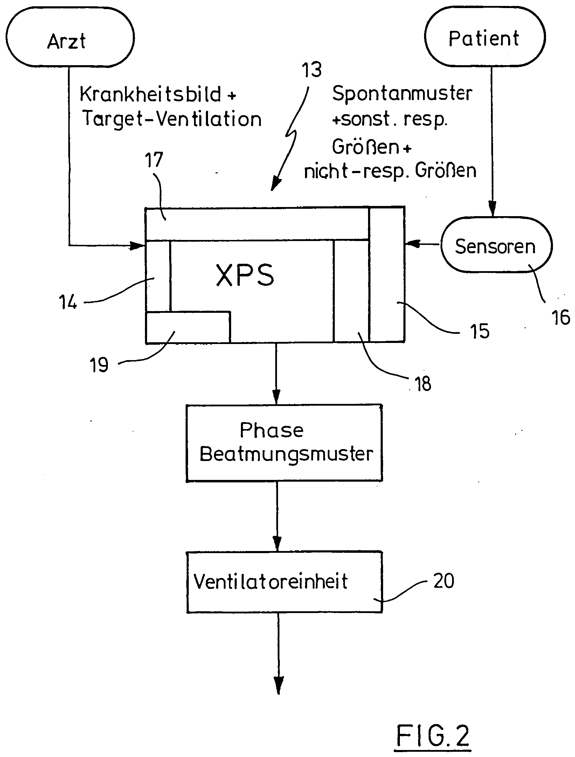

Fig. 2 zeigt den prinzipiellen Aufbau der steuerungstechnischen Vorrichtungskomponenten. Eine Steuereinheit (13) ist mit einem Eingabemodul (14) zur Dateneingabe versehen. Über das Eingabemodul (14) können beispielsweise von einem Arzt Daten zum Krankheitsbild sowie zur Target-Ventilation eingegeben werden. Ein Analysator (15) ist mit Sensoren (16) verbunden, die mindestens einen Beatmungsparameter eines Patienten erfassen.

Die Steuereinheit (13) weist darüber hinaus einen Sollwertspeicher (17) sowie einen Transformator (18) auf, der vom Analysator (15) bereitgestellte Daten in Sollwerte für ein Beatmungsmuster umrechnet. Darüber hinaus ist die Steuereinheit (13) mit einem Koordinator (19) versehen, der eine Aktivierung eines jeweiligen Beatmungsmusters in Abhängigkeit von den im Bereich des Sollwertspeichers (17) abgelegten Steuerwerten durchführt.

In Abhängigkeit von den im Bereich des Sollwertspeichers (17) abgelegten Daten gibt die Steuereinheit (13) eine Phasenlage sowie ein Beatmungsmuster für eine Atemgaspumpe (20) vor, die typischerweise innerhalb des in Figur 1 dargestellten Gerätegehäuses (1) angeordnet ist.

Eine Durchführung des Verfahrens erfolgt typischerweise derart, daß zunächst in einer Analysephase ein Flowmuster des Patienten entweder bei Umgebungsdruck, einem geringen Druck bei der Durchführung des CPAP-Verfahrens oder bei einem geringen Druck bei einer assistierten Bi-Level-Beatmung durchgeführt wird. Die Berechnung des Beatmungsmusters kann beispielsweise entsprechend der Berechnungsvorschrift

$$P(t)=Resistance*Flow(t)+Elastance*Volumen(t)+PEEPintr.$$

erfolgen. Darüber hinaus ist es möglich, komplexere Berechnungen durchzuführen, bei denen der zeitliche Verlauf der erfaßten Meßgrößen und/oder Informationen hinsichtlich des Patientenzustandes berücksichtigt werden. Eine Verarbeitung der Daten kann beispielsweise unter Verwendung eines Expertensystems erfolgen. Die Vorgabe des Beatmungsmusters kann sowohl in Form eines Druckmusters als auch in Form eines Flowmusters erfolgen. Nach einer Errechnung des vorgegebenen Beatmungsmusters erfolgt ein Übergang in die mandatorische Beatmung in einem kontinuierlichen Übergangsprozeß und somit sanft.

Dies bedeutet, daß das berechnete Beatmungsmuster als Sollwert nicht sofort vollständig und ausschließlich zur Gerätesteuerung verwendet wird, sondern daß innerhalb einer Übergangsphase eine Annäherung an das Sollwertmuster erfolgt. Beispielsweise ist es möglich, ein auf einer Druckkontrolle basierendes Beatmungsmuster durch langsame Steigerung der maximalen Druckamplitude pro Atemzug über einem definierten Zeitraum auf das zuvor berechnete Zielniveau zu verschieben. Ebenfalls ist es denkbar, das berechnete Beatmungsmuster mit einem Standardmuster zu mischen. Das Standardmuster kann beispielsweise als Rechteckmuster oder als Rampe realisiert sein. Insbesondere ist auch daran gedacht, daß Einsetzen der Beatmungssteuerung synchron zu einem vorbestimmten Rhythmus durchzuführen.

Nach dem Erreichen einer vollständigen mandatorischen Steuerung des Beatmungsgerätes erfolgt über die Atemgaspumpe (20) eine vollständige Vorgabe sowohl der Form als auch des Taktes und der Geschwindigkeit der jeweiligen Zielgröße, nämlich des Druckes oder des Flows.

Gemäß einer Ausführungsform ist es möglich, eine automatische Aktivierung einer Analysephase während der Durchführung der mandatorischen Gerätesteuerung in Abhängigkeit von einer Meßwertauswertung durchzuführen. Insbesondere kann eine derartige Meßwertauswertung derart erfolgen, daß eine Spontanatmung beliebigen Verlaufes des Patienten detektiert wird. Kriterien für eine entsprechende Spontanatmungserkennung können beispielsweise in einem expertenbasierten Regelwerk abgespeichert sein. Die Auswertung kann auch unter Verwendung einer Fuzzy-logic erfolgen. Als Eingangsgrößen für dieses Regelwerk können die gemessenen respiratorischen und nicht respiratorischen Größen verwendet werden, die dann mit entsprechenden Schwellwerten für Absolutwerte oder für entsprechende Streubreiten verglichen werden.

Der betreffende Übergang von der mandatorischen Gerätesteuerung in die Analysephase erfolgt dann, wenn eine vorgegebene Schwellwerte übersteigende Eigenaktivität des Patienten detektiert wird. Eine derartige Detektion kann beispielsweise durch die Auswertung einer Kennung für Inspirationsphasen und Expirationsphasen erfolgen. Wird hierbei festgestellt, daß über einen vorgegebenen Zeitraum ein Phasenversatz vorliegt, so stellt dies einen Indikator für eine erforderliche Musteradaption dar. Es erfolgt dann ein Übergang der Gerätesteuerung in die Analysephase, um ein aktuelles angepaßtes Beatmungsmuster für den Sollwertspeicher (17) der Steuereinheit (13) zu berechnen.

Ein besonderer Gesichtspunkt der Gerätesteuerung ist in der Durchführung einer Synchronisation zu sehen. Es werden zunächst extern Zielwerte für die Beatmung, beispielsweise die Beatmungsfrequenz und das Atemzeitverhältnis vorgegeben. Der Patient wird dann vom Gerät zunächst mit seiner Spontanfrequenz oder eine leicht darüber liegenden Frequenz beatmet und anschließend langsam und unter Überwachung der Eigenaktivität mit der Zielfrequenz synchronisiert. Entwikkelt der Patient während des Synchronisationsvorganges beispielsweise durch ein Absenken der Atemfrequenz erneut eine Eigenaktivität, so erfolgt erneut eine Korrektur der Atemfrequenz in Richtung der ursprünglichen Spontanfrequenz. Die Vermeidung einer Eigenaktivität des Patienten und somit eine möglichst vollständige Entlastung des Patienten von Atemarbeit hat somit bei der Gerätesteuerung gegenüber dem Erreichen des extern vorgegebenen Zielwertes eine höhere Priorität. Alternativ oder ergänzend zur Vorgabe von Atemfrequenz und Atemzeitverhältnis ist es auch möglich, extern ein Tidalvolumen zu definieren.

Gemäß einer weiteren Ausführungsform der Erfindung wird in einer Analysephase zunächst der Rhythmus der Atmung, d. h. die Atemfrequenz und das Atemzeitverhältnis bzw. die konkreten Dauern der Inspirationszeit sowie der Expirationszeit bestimmt. Anschließend wird von der Gerätesteuerung phasenrichtig und synchronisiert zur Spontanaktivität eine mandatorische Steuerung der Beatmung unter Verwendung eines Standardmusters vorgegeben. Als Standardmuster können beispielsweise Rechteckverläufe, Rampenverläufe, Parabelverläufe oder sinusförmige Verläufe definiert sein.

## Patentansprüche

1. Vorrichtung zur Beatmung, die eine Atemgaspumpe (20), eine Steuereinheit (13) für die Atemgaspumpe (20) sowie einen Anschluss zur Verbindung mit einem Beatmungsschlauch aufweist und bei der die Steuereinheit (13) mit einem Sollwertspeicher (17) für mindestens einen Steuerparameter gekoppelt ist, der zu einem Beatmungsmuster korrespondiert, sowie bei der die Steuereinheit (13) mit einem Analysator (15) gekoppelt ist, der mit mindestens einem Sensor (16) für mindestens einen Atemparameter verbunden ist, und bei der die Steuereinheit (13) einen Transformator (18) zur Umrechnung von Daten des Analysators (15) in Vorgabewerte für den Sollwertspeicher (17) aufweist und bei der die Steuereinheit (13) einen Koordinator (19) zur Aktivierung einer Ansteuerung der Atemgaspumpe (20) in Abhängigkeit von den errechneten und den im Sollwertspeicher abgelegten Steuerparametern einer Drucksteuerung aufweist, **dadurch gekennzeichnet, dass** der Analysator (15) den Flowverlauf und die Atemfrequenz und das Verhältnis von Inspirationsphasen zu Expirationsphasen analysiert und dass daraus der Steuerparameter als Druckmuster berechnet wird, sowie dass die Steuereinheit (13) zur automatischen Einleitung einer Analysephase während der Durchführung der mandatorischen Beatmung als Reaktion auf eine Spontanatmung des Patienten ausgebildet ist.

## Claims

1. Ventilator, comprising a respiratory gas pump (20), a control unit (13) for the respiratory gas pump (20) and a connector for connecting to a ventilation tube, and wherein the control unit (13) is coupled to a setpoint value memory (17) for at least one control parameter, which corresponds to a ventilation pattern, and wherein the control unit (13) is coupled to an analysing means (15), which is connected to at least one sensor (16) for at least one respiration parameter, and wherein the control unit (13) comprises a transforming means (18) for converting data of the analysing means (15) into prescription values for the setpoint value memory (17) and wherein the control unit (13) comprises a coordination means (19) for activating an actuation of the respiratory gas pump (20) as a function of the control parameters, both calculated and saved in the setpoint value memory, of a pressure controller, **characterized in that** the analysing means (15) analyses the flow curve and the respiratory frequency and the ratio of inspiration phases to expiration phases and **in that** the control parameter is calculated as a pressure pattern therefrom, and **in that** the control unit (13) is embodied to automatically introduce an analysis phase while implementing the mandatory ventilation as a reaction to spontaneous respiration of the patient.

## Revendications

1. Dispositif servant à la ventilation, lequel possède une pompe à gaz respiratoire (20), une unité de commande (13) pour la pompe à gaz respiratoire (20) ainsi qu'un raccord servant à la liaison avec un tuyau de ventilation et avec lequel l'unité de commande (13) est connectée à une mémoire de valeurs de consigne (17) pour au moins un paramètre de commande, lequel correspond à un module de ventilation, et aussi avec lequel l'unité de commande (13) est connectée à un analyseur (15) qui est relié à au moins un capteur (16) pour au moins un paramètre respiratoire, et avec lequel l'unité de commande (13) possède un transformateur (18) destiné à convertir des données de l'analyseur (15) en valeurs prédéfinies pour la mémoire de valeurs de consigne (17) et avec lequel l'unité de commande (13) possède un coordinateur (19) destiné à l'activation d'une excitation de la pompe à gaz respiratoire (20) en fonction des paramètres de commande d'une commande de pression calculés et stockés dans la mémoire de valeurs de consigne, **caractérisé en ce que** l'analyseur (15) analyse la courbe de débit et la fréquence respiratoire et le rapport entre les phases d'inspiration et les phases d'expiration et **en ce que** le paramètre de commande est calculé à partir de cela en tant que modèle de pression, et **en ce que** l'unité de commande (13) est configurée pour initier automatiquement une phase d'analyse pendant la réalisation de la ventilation contrôlée en tant que réaction à une respiration spontanée du patient.

FIG.1

FIG.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1346743 A
- EP 1129742 A
- WO 0100265 A
- US 6349724 B
- DE 10014427 A1